# EUROPEAN PATENT APPLICATION

(11) **EP 1 054 259 A1**
(43) Date of publication of application: **22.11.2000**
(21) Application number: 99870106.4
(22) Date of filing: 19.05.1999
(51) Int. Cl.: G01N 33/543, C12Q 1/68

(54) **Method for the identification of a target compound**

(71) Applicant: Remacle, José, 5020 Malonne (BE)
(72) Inventor: Remacle, José, 5020 Malonne (BE); Zammatteo, Nathalie, 5100 Jambes (BE); Alexandre, Isabelle, 5170 Lesve (BE); Hamels, Sandrine, 6280 Loverval (BE); Houbion, Yves, 5150 Floreffe (BE); De Longueville, Françoise, 5100 Jambes (BE)
(74) Representative: Van Malderen, Joelle

(57) **Abstract**

The present invention is related to a method for the identification and/or the quantification of a target compound obtained from a sample, preferably a biological sample, comprising the steps of:
- putting into contact the target compound with a capture molecule in order in order to allow a specific binding between said target compound with a capture molecule, said capture molecule being fixed upon a surface of a solid support according to an array comprising a density of at least 20 discrete regions per cm², each of said discrete regions being fixed with one species of capture molecules,
- performing a reaction leading to a precipitate formed at the location of said binding,
- determining the possible presence of precipitate(s) in discrete region(s), and
- correlating the presence of the precipitate(s) at the discrete region(s) with the identification and/or a quantification of said target compound.

## Description

### Field of the invention

The present invention is related to a method for the identification and/or the quantification of a target compound obtained from a biological sample by binding to a capture molecule fixed upon chips.

The present invention is also related to an identification and/or quantification device based upon said method, that allows the identification and/or the quantification of positive locations of bounded target compounds upon said chips.

### Background on the invention and state of the art

Biological assays are mainly based upon interaction specificity between two biological molecules such as the binding of two strands of nucleic acid molecules, the binding between an antigen and an antibody or the binding between a ligand and its receptor. The present challenge of biological assays is to perform simultaneously the multiple detection of molecules present in a sample. Miniaturisation and development of arrays upon the surface of "biochips" are tools that allow multiplex reactions in a microscopic format, said detection being made with a limited volume of sample for the screening and/or the identification of multiple possible target compounds. These arrays are formed of discrete regions, each one containing a specific capture molecule used for the binding of the target compound. These discrete regions, as small as a few micrometers, allow the fixation of several thousands capture molecules per cm² surface.

However, the detection of bounded target compounds is difficult, since their amount is very small due to said miniaturisation. Typically, the amount of bounded target compounds present in the sample to be detected is usually few fentomoles or even few attomoles. Therefore, only extremely sensitive methods are adequate for such detection.

It has been proposed a labelling of a target compound like DNA with fluorescent molecules after their possible genetic amplification (by PCR, LCR, etc.). When an RNA molecule has to be detected, said nucleic acid is first transformed into a cDNA before its possible amplification. If direct labelling of the target compound is not possible, a double reaction (sandwich reaction) can be performed. However, the amount of fluorescent molecules is so low that it is necessary to develop specific array scanners for the detection and/or the quantification of the bounded compound upon the "hybridisation chips". Said expensive specific scanners comprise a laser scanner for excitation of the fluorescent molecules, a pinhole for decreasing the noise fluorescent background, and a photomultiplier for increasing the sensitivity of the detection.

Alternative detection methods that present a high sensitivity are described in the documents US-5,821,060 and WO95/04160 and are based upon the detection using expensive devices such as mass spectrometers.

It has also been proposed methods based upon precipitation of specific products that are the result of enzymatic activities (WO90/06372, EP-A-0444649, EP-A-0124124). However, said methods are either characterized by a low sensitivity or are not adequate for the detection of a target compound upon "hybridisation chips", because the precipitate will occur at a certain distance of the reaction binding and its location can not be easily correlated with a specific bounded target compound. In addition, the density of the precipitate of such enzymatic reactions is not enough opaque for allowing a detection by light absorption.

It has also been proposed to improve the detection by fixing a soluble product obtained from the enzymatic reaction with a metal before its precipitation. However, as the result of said enzymatic reaction is a soluble product, there is no correlation between the location of the precipitate and the detection of a specific bounded target compound.

### Aims of the invention

The present invention aims to provide a new identification and/or quantification method of one or more target compounds present (possibly simultaneously) in a biological sample and that will not present the drawbacks of the state of the art.

The present invention aims to provide such a method that is simple and not expensive, that allows the detection of said target compounds by using fixed capture molecules upon arrays of the surface of a solid support.

A last aim of the present invention is to provide also a simple and non-expensive device based upon said method, that improves the identification and/or the quantification of bounded target compounds upon "hybridisation chips".

### Summary of the invention

The present invention is related to a method for identification and/or quantification of at least one target compound present in a biological sample by its binding upon a capture molecule fixed upon arrays of a solid support (hereafter called "hybridisation chips"), the binding of said target compound upon its corresponding capture molecule resulting in the formation of a metal precipitate at the location of said capture molecule.

Advantageously, said method comprises the steps of:
- putting into contact the target compound with a capture molecule in order to allow a specific binding between said target compound with a (corresponding) capture molecule, said capture molecule being fixed upon a surface of a solid support according to an array comprising at least a density of 20 discrete regions per cm², each of said discrete regions being fixed with one species of capture molecules,
- performing a reaction, preferably a catalytic reaction, leading to a formation of a precipitate at the location of said binding,
- determining the possible presence of a precipitate in a discrete region, and
- correlating the presence of the precipitate(s) at the discrete region(s) with the identification and/or a quantification of said target compounds in the biological sample.

The "hybridisation chips" according to the invention are any kind of solid support that allow the formation of arrays of capture molecules upon one or more of its surfaces. Said solid support can be made of glasses, filters, electronic device, polymeric or metallic materials, etc. Preferably, said arrays contain specific locations, each of them containing normally only one species of capture molecule.

The biological target compounds according to the invention may be present in a biological (or possibly a non-biological) sample such as clinical samples extracted from blood, urine, faeces, saliva, pus, serum, tissues, fermentation solutions or culture media. Said target compounds are preferably purified and/or amplified if necessary by known methods by the person skilled in the art before their detection and/or quantification upon the "hybridisation chips".

Preferably, the formation of a precipitate at the location of the binding is obtained with the fixation of a metallic compound upon the bounded target compound or by the result of a reduction of a metal in the presence of an enzyme. For instance, a reduction of silver in the presence of colloidal gold allows the formation of a precipitate at a distance not exceeding few micrometers from the bounded target compound to its capture molecule.

According to the invention, the specific locations on the array are smaller than 1000 µm in length. These locations or sports have preferably a diameter comprised between 10 and 500 µm and are separated by distance of similar order of magnitude so that the array of the solid support comprises between 100 and 250,000 spots upon the surface of 1 cm². However, it is also possible to prepare spots smaller as 1 µm or less upon which the capture molecules are fixed. The formation of said spots or locations would be obtained by known microelectronic or photolitographic processes and devices that allow the fixation of said capture molecules on the surface of the solid support either by a covalent linkage or a non-covalent adsorption. The covalent linkage technique is preferred in order to control specifically the sites of capture molecules fixation and avoid possible drawbacks that may result with several capture molecules like nucleic acids or antibodies that can be desorbed during incubation or washing step.

One of the preferred embodiment is the fixation of biological molecules like proteins, peptides or nucleic acids by linkage of amino groups on activated glass bearing aldehyde moiety. The incorporation of an amine group in the nucleic acid chain is easily obtained using aminated nucleotide during their synthesis. Aminated amino acids can be fixed upon the surface of a solid support like glass bearing aldehyde groups as described by Schena et al. (Proc. Natl. Acad. Sci. USA, **93**, pp. 10614-10619 (1996)) or as described in the document US-5,605,662 and the publication of Krensky et al. (Nucleic Acids Research, **15**, pp. 2891-2909 (1987)). The linkage between an amino and a carboxyl group is obtained by the presence of a coupling agent like carbodiimide compounds as described by Joos et al. (Anal. Biochem., **247**, pp. 96-101 (1997)). Thiol modified oligonucleotides can be used also to obtain a reaction with amino groups upon the surface of a solid support in the presence of cross-linking molecules (Thrisey et al., Nucleic Acids Research, **24**, pp. 3031-3039 (1996)). Similarly, oligonucleotides can be fixed to a gel like polyacrylamide bearing hydroxyl and aldehyde groups as described in the document US-5,552,270 and WO98/28444.

The fixation of DNA strands on proteins thereafter specifically attached to sites specific locations on a substrate, is described in the document US-5,561,071. It is also known that capture chemicals can be linked to microtubes that are then spatially arranged in order to produce an array, as described in the document GB-3 319 838, or to obtain the direct synthesis of oligonucleotides on specific surfaces by using photolithographic techniques as described in the documents EP-0476014, US-5,510,270, US-5,445,934, W097/29212, US-5,605,662, US-5,632,957 and W094/22889.

All these methods for the fixation of capture molecules on the surface of a solid support in order to obtain the above-described arrays are compatible with the present invention.

The binding (or recognition) of the target compound upon their corresponding specific capture molecules is a spontaneous non-covalent reaction when performed in optimal conditions. It involves non-covalent chemical bindings. The medium composition and other physical and chemical factors affect the rate and the strength of the binding. For example for nucleotide strand recognition, low stringency and high temperature lower the rate and the strength of the binding between the two complementary strands. However, they also very much lower the non specific binding between two strands which are not perfectly complementary so that a compromise is always looked for in order to obtain the highest specific with the lowest non specific binding. When several sequences are similar, the specificity of the binding can be enhanced by addition of a small amount of non-labelled molecules, which will compete with their complementary sequence but much more with the other ones, thus lowering the level of cross-reactions.

The optimisation of the binding conditions is also necessary for antigen/antibody or ligand/receptors recognition, but they are usually rather specific.

A preferred embodiment of this invention is to take party of the amplification given by the catalytic reduction of Ag⁺ in the contact of other metals like gold. Gold nanoparticules are currently available and they can be easily fixed to molecules like protein. For example, streptavidin coated gold particles are available on the market.

According to a preferred embodiment of this invention, one uses a labelled target molecule, which is then recognised by a conjugate. This labelled molecule can be considered as a first member of the binding pair. Biotin is a good candidate as label for the target but haptens are also possible. For DNA, the labelling is easily done by incorporation of biotinylated nucleotides during their amplification. For the RNA, biotinylated nucleotides are used for their copy in cDNA or thereafter in the amplification step. Amplification of the nucleotide sequences is a common practice since the target molecules are often present in very low concentrations. Proteins are easily labelled using NHS-biotin or other reactions. Once the biotinylated molecules are captured, a streptavidin-gold complex, which is the second member of the binding pair, is added and the streptavidin specifically recognises biotin, so that the complex is fixed at the location where the target is fixed. If haptens are used as labelled, then an antibody-gold complex will be used. Then a reactive mixture containing Ag⁺ and a reducing agent is added on the surface and Ag will deposit on the gold particles leading to the formation of crystal particles.

Direct labelling of the target molecules with gold is possible using gold-labelled antigens, antibodies or nucleotides but these would have to be synthesised.

An alternative is to avoid any labelling of the target molecule, and then a second probe is used which is labelled. This can be useful for DNA but also when antigen or antibodies are used in the reaction. They then formed a sandwich hybridisation or a sandwich reaction with the capture molecule fixing the target and the labelled probe, which allows the detection to go on. Like above, the labelled probe is able to catalyse itself the precipitation of the metal or it does it through a second complex.

The Ag precipitation corresponds to the location of the biotinylated probe. At said location is well defined, it is possible to identify the presence of said precipitate and to attribute the value to the presence or not of the specific target molecule on a specific spot of the array.

The precipitate has the form of small crystals that reach with time a diameter of about 1 µm. The formation of these small crystals represents a real amplification of the signal since they originated from the presence of gold particles a few nm in diameter.

Unexpectedly, within a given range of labelled probes present on the surface, a concentration curve could be obtained between the gold-labelled probe concentration and the amount of precipitate on the surface. One constraint of the array is that the detection signal has to be correlated with the location where it originates.

Because of its granular form, the precipitate modifies the reflection of the light. It also leads to a strong diffusion of the light (spot detected), which is recordable by known detection means. One unexpected observation is that this assay for the presence of silver crystals was found very sensitive, probably due to the fact that the crystal size is in the same order of magnitude as the wavelength of the light so that its interference is very high. Such diffusion assays are typically detected and recorded from the reflection of a light beam with photodiodes.

The fact that the silver precipitate appears as a black surface also allows to use a scanner, which assays for the absorption of the light through the transparent surface of the array. The presence of insoluble precipitate will absorb the light, which is then detected and recorded. The advantage of the scanner is that only a small portion of the array is detected at one time so that a much better resolution can be obtained. Either the illumination beam or the detection surface is focalised and the signal is recorded so that the image of the array can be reconstituted. The detection means (detector) can be a CCD, which measures the overall array. The resolution of the detection is then dependent on the number of pixels of the camera. On the other end, the detector can be constituted of photo-diodes arranged into a line and the image is scanned by moving in front of this line. Scanners with a sensitivity of 11 µm for a pixel can be constructed, which are sufficient to analyse spots of 50 µm in diameter or bigger.

A full illumination of the array combined with a recording of the light transmitted is also possible. Its advantage is to be faster than the scanner, but seems to be less sensitive.

As a metal, silver is able to reflect light by itself. Even if the efficiency of this reflection is low, it exists and can be used in order to localise the silver precipitate (spots). Because if its metal nature, other methods like variations of an electromagnetic field or electric conductance are also possible.

Another aspect of the present invention concerns a diagnostic and/or quantification apparatus of one or more identical or different target compound(s) obtained from a sample, said device comprising:
- a detection device of precipitates (spots) upon the surface of a solid support resulting from the binding of a target compound upon its corresponding capture molecule above-described,
- possibly a reading device of information(s) recorded upon said solid support (such as barcodes), and
- a computer programmed to:
   - possibly recognise the discrete regions bearing capture molecules,
   - collect the results obtained from said detection device, possibly correlated with the information(s) obtained from said reading device, and
   - carry out a diagnostic and/or quantification of said target compound(s).

The present invention is also related to a computer program product (software), characterized in that it comprises means to collect the results obtained from said detection device and possibly the information(s) obtained by said reading device, and means to carry out a diagnostic and/or quantification of a specific target compound resulting from the analysis of said results, possibly correlated to the read information(s).

Said means of this computer program product are able to obtain a discrimination between the spots and a possible detected background noise, for instance by the identification of homogeneous parts of an image after having been merged into two classes used as training sets. This discrimination can be enhanced by post-classification contextual filters techniques.

Said means are also able to identify the contour of the spot itself, which will be superposed to the original image and will allow the measure of intensity level of the counted pixels identified in the spot.

The quantification means allow an integration of all pixels intensity present in the spot or a recording the overall level of intensity of the homogeneous parts of the spot.

Furthermore, these means allow a statistical comparative analysis between the spots of each sample and a control (standard target compound) or between two or more spots (preferably with a correlation with the recorded informations of the solid support). Image correlation could be obtained between the spot image and said standard target compound spot image in order to discriminate spots that are statistically different in one test compared to another.

The recorded signal(s) by the detection device and the reading device can be read, processed as electronically computerised data, analysed by said appropriate computer program product (software).

According to a specific embodiment of the present invention, the array bears fixed oligonucleotide capture probes so as to allow a detection, amplification and possibility quantification of nucleic acid sequences upon a same solid support. In an alternative form of execution, the array comprises fixed PCR primers in order to obtain the production of amplicons and fixation of amplicons upon the surface according to the method described by Rasmussen et al. (Anal. Biochem., **198**, pp. 138-205 (1991)), which allows thereafter their detection.

The array according to this invention is used in a diagnostic kit, in a diagnostic and reading device which allows automatic lecture of the sample preparation containing chemical or biological compounds, possibly after a previous treatment of said chemical or biological compound, such as genetic amplification of a nucleotide sequence.

Preferably, the detection and/or quantification device according to the invention is a system that combines multiple steps or substeps within an integrated system as an automatic nucleic acid diagnostic system, which allows the steps of purification of the nucleic acid sequences in a sample, their possible amplification (through known genetic amplification methods), their diagnostic and possibly their quantification.

Preferred embodiments of the present invention will be described in the following non-limiting examples.

### Description of the drawings

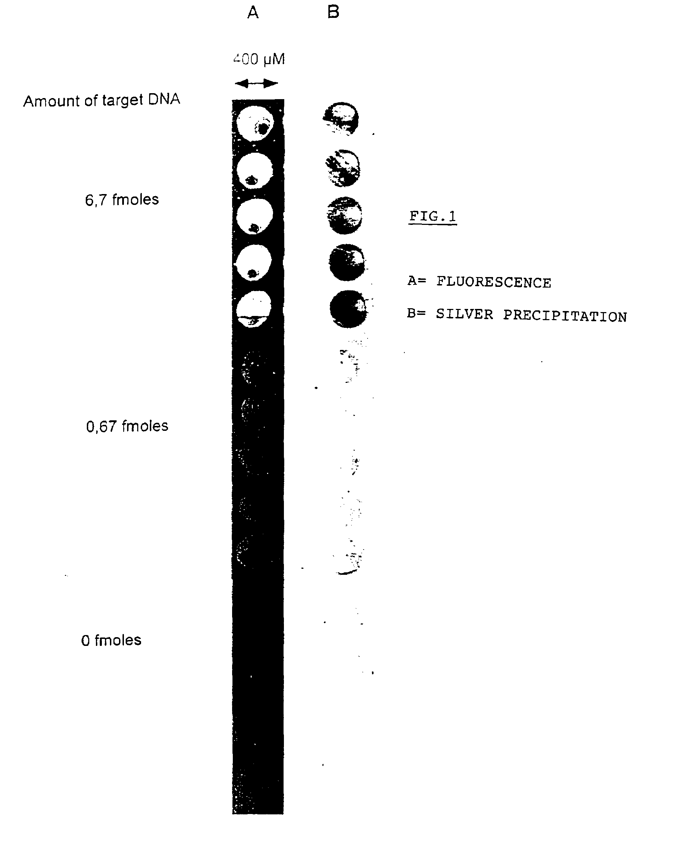

- Figure 1: compares the detection of target molecules obtained on arrays composed of DNA capture probes covalently fixed on the glass and used to detect 3 concentrations of biotinylated target DNA either in fluorescence or after silver concentration.

### Detection of DNA on biochips

In this experiment, target DNA labelled is detected by direct hybridisation on capture probes bound to the array. Capture probes were covalently bound on glass and direct hybridisation performed with complementary biotinylated DNA. The positive hybridisation was detected with silver precipitate catalysed by the nanogold particles linked to streptavidin.

### Binding of capture probes on glass

Activated glass bearing aldehyde groups were purchased from CEL Associates (USA). Aminated capture probes for CMV DNA were constructed by PCR amplification of the DNA using aminated primer as described by Zammatteo et al. (Anal. Biochem., **253**, pp. 180-189 (1997)). The primers were purchased from Eurogentec (Liège, Belgium). Quantification of the amplicons was done by their absorption at 260 nm.

For the grafting on glass, a solution of aminated amplicons at 0.2 µm in MES 0.1 M pH 6.5 was first heated at 100 °C for 5 min and then spotted by a robot using 250 µm diameter pins (Genetix, UK). After incubation of 1 h at 20 °C, they were washed with SDS solution at 0.1% and then two times with water. They were then incubated with NaBH₄ at 2.5 mg/ml solution for 5 min then washed in water and heated at 95 °C for 3 min before being dried.

### Hybridisation of the target molecule

The target molecule was obtained by amplification by PCR in the presence of biotinylated dUTP at 1 mM (Alexandre et al., Biotechniques, **25**, pp. 676-683 (1998)). Plasmids containing the sequence of CMV virus were used for the PCR. After amplification, the PCR products were purified using a kit of high pure PCR product purification (Boehringer, Mannheim, Germany) and quantified by ethidium bromide staining after separation on a 2% agarose gel.

For the hybridisation, various concentrations 0.67, 6.7 and 67 fm in 5 µl of biotinylated target DNA were added in a SSC 2X Denhard solution containing 20 µg of Salmon DNA. A drop of this solution (5 µl) was added on the array and incubated for 2 h at 65 °C in a wet atmosphere. The array was then washed 4 times with a maleic acid buffer 10 mM pH 7.5, containing NaCl 15 mM and Tween 0.1%.

### Silver precipitation on the array after silver precipitation

The array was first incubated for 45 min with 0.8 ml of a streptavidin-colloidal gold (Sigma) diluted 1,000 times in a maleic buffer 150 mM pH 7.4 containing NaCl 100 mM and 0.1% dry milk ponder. The arrays were then washed 5 times 2 min in the maleic acid buffer 10 mM pH 7.4 containing 15 mM NaCl and Tween 0.1%. A "silver enhancement reagent" (40 µl) from Sigma was added onto the array and changed after 10 and then 5 min. After washing in the maleic buffer, the array was dried.

### Detection and analysis of the array

The array was scanned and the digitalised image was treated with form recognition software in order to delimitate and identify the spots. The level of the pixels of each spot was integrated and a value given to each spot. The values were corrected for the background obtained in three places where no capture probes have been fixed.

### Detection of proteins on biochips

### Fixation of antibodies on the array

The glass of the array was activated as described here above in order to obtain aldehyde groups on the surface. The antibodies used in this experiment were raised against bovine serum albumin for positive control and non specific IgG for negative control. The antibodies at 10 µg/ml in PBS solution were spotted using the 250 µm diameter pins directly on the glass. The amino groups of the antibodies could react with the aldehyde present on the glass. The reaction was performed for 1h at room temperature. The glasses were washed with a PBS buffer.

### Detection of bovine serum albumin by ELISA on the array

A solution of bovine serum albumin (BSA) at 10 µg/ml in PBS containing 0.1% casein was added on the array and incubated for 30 min. The array was then washed 3 times with PBS containing 0.1% Tween 20 and then incubated with a solution of biotinylated anti-BSA at 20 µg/ml in PBS containing 0.1% casein. The incubation was performed for 30 min. A streptavidin-Gold complex at 1 µg/ml was then incubated for 30 min in a PBS solution containing 0.1% casein. The presence of gold served as a center for silver reduction. The silver precipitation was performed with a "silver enhancement reagent" from Sigma with a change of the solution after 10 min and then again after 5 min. The glasses were then scanned and the data analysed as presented in the example here above.

## Claims

1. A method for the identification and/or the quantification of a target compound obtained from a sample, preferably a biological sample, comprising the steps of:
- putting into contact the target compound with a capture molecule in order in order to allow a specific binding between said target compound with a capture molecule, said capture molecule being fixed upon a surface of a solid support according to an array comprising a density of at least 20 discrete regions per cm², each of said discrete regions being fixed with one species of capture molecules,
- performing a reaction leading to a precipitate formed at the location of said binding,
- determining the possible presence of precipitate(s) in discrete region(s), and
- correlating the presence of the precipitate(s) at the discrete region(s) with the identification and/or a quantification of said target compound.

2. Method according to claim 1, characterized in that the reaction leading to the formation of a precipitate is obtained by the deposit of a metallic compound upon the bounded target compound.

3. Method according to claim 2, characterized in that said metallic compound is a magnetic metallic compound.

4. Method according to any one of the preceding claims, characterized in that the reaction leading to the formation of the precipitate is a reduction of a metal in the presence of an enzyme.

5. Method according to claim 1 or 2, characterized in that the reaction leading to the formation of the precipitate is a chemical reduction of silver in the presence of colloidal gold coupled to the bounded target compound.

6. Method according to any one of the preceding claims, characterized in that the specific binding between the target compound and its corresponding capture molecule is an hybridisation between two nucleotide sequences.

7. Method according to any one of the claims 1 to 5, characterized in that the binding between the target compound and its corresponding capture molecule is a reaction between an antigenic structure and its corresponding antibody.

8. Method according to any one of the preceding claims, characterized in that the binding between the target compound and its corresponding capture molecule is a reaction between a receptor and its corresponding ligand.

9. Method according to any one of the preceding claims, characterized in that the possible presence of a precipitate is obtained by reflection, absorption or diffusion of a light beam, preferably a laser beam, upon said precipitate.

10. Method according to any one of the preceding claims, characterized in that the presence of a precipitate in a discrete region is obtained by variation of an electromagnetic field or the conductance of an electric current.

11. Diagnostic and/or quantification apparatus of one or more identical or different target compound(s) obtained from a sample, characterized in that it comprises:
- a detection device of precipitate(s) formed at the location of a binding of said target compound with a capture molecule upon a surface of a solid support according to an array comprising at least 20 discrete regions per cm², each of said discrete regions being fixed with one species of capture molecule,
- possibly a reading device of information(s) recorded upon said solid support (such as barcodes), and
- a computer programmed to:
- possibly recognise discrete regions bearing capture molecules,
- collect the results (formation of a precipitate at a specific location) obtained from said detection device, and possibly the information(s) obtained from said reading device, and
- carry out a diagnostic and/or quantification of said target compound(s).

12. Computer program product characterized in that it comprises means for collecting the results obtained from the detection apparatus according to claim 11 and possibly the information(s) obtained from the reading device of the diagnostic and/or quantification apparatus according to claim 11, to carry out a diagnostic and/or quantification of a target compound by said diagnostic and/or quantification apparatus.
